# EUROPEAN PATENT APPLICATION

(11) **EP 2 700 407 A1**
(43) Date of publication of application: **26.02.2014**
(21) Application number: 12774148.6
(22) Date of filing: 18.04.2012
(51) Int. Cl.: A61K 36/18, A61K 9/06, A61K 9/50, A61K 31/05, A61K 31/192, A61K 47/36, A61K 47/38, A61P 1/00, A61P 1/04, A61P 3/00, A61P 31/04

(54) **GRANULES IN LIQUID DOSAGE FORM**

(30) Priority: 18.04.2011 JP 2011092502; 13.01.2012 JP 2012005416
(71) Applicant: Idemitsu Kosan Co., Ltd, Tokyo 100-8321 (JP)
(72) Inventor: ITO, Shinji, Sodegaura-shi Chiba 299-0293 (JP); OOIWA, Seika, Sodegaura-shi Chiba 299-0293 (JP)
(74) Representative: Vossius & Partner
(86) International application number: PCT/JP2012/060452
(87) International publication number: WO 2012/144519

(57) **Abstract**

A formulation which comprises coated granules and a water-soluble gel, wherein the coated granules comprise an oil absorbent which comprises cashew nut shell liquid, anacardic acid, cardanol, or cardol, and wherein the coated granules are suspended in the water-soluble gel.

## Description

### TECHNICAL FIELD

The present invention relates to a formulation which improves the handleability of granules containing cashew nut shell liquid (CNSL) and delays the release of the cashew nut shell liquid from the granules.

### BACKGROUND ART

Cashew nut shell liquid is oily liquid contained in the shell of the seed of a cashew nut obtained from the cashew nut tree (*Anacardium occidentale L.).* The cashew nut shell liquid mainly contains, as the components thereof, anacardic acid, cardanol, cardol and methylcardol.

A method for preparing the cashew nut shell liquid includes a heating process and a solvent extraction process. Usually, the cashew nut shell liquid is heat-treated by the producer of cashew nuts to convert the anacardic acid into cardanol for use.

The cashew nut shell liquid has the effect of causing a rash and thus requires care in handling. The rash can be caused by any of the main components of the anacardic acid, the cardanol, and the cardol. Thus, there has been a need for the development of a method for safely handling the cashew nut shell liquid.

Uses of the industrial product include a novel polyether polyol prepared by alkoxylation of renewable resource materials, and particularly cashew nut shell liquid (Patent Document 1); an antimicrobial polylactic acid based resin composition containing a polylactic acid based resin and carboxyalkylated cashew nut shell liquid (Patent Document 2); and a fibrous nanoscale self-aggregate using cardanol (Patent Document 3). In any of these patents, heated cashew nut shell liquid is used.

Some examples of the use in livestock have been reported. Uses of unheated cashew nut shell liquid and anacardic acid, which is a component of the cashew nut shell liquid, as a feed have been reported, including a coccidiostat composition containing cashew nut shell liquid and/or anacardic acid, which is a main component of the cashew nut shell liquid (Patent Document 4); a coccidiostat containing cashew nut shell liquid and/or anacardic acid, and at least one selected from organozinc compounds, betaines, and *Bacillus* sp. as active ingredients; and a feed for prevention and/or treatment a disease caused by *Coccidia* (Patent Document 5); and an agent for relieving a disease caused by *Coccidia* containing cashew nut shell liquid and/or anacardic acid as an active ingredient and a feed containing the agent (Patent Document 6).

The prior art documents, however, do not describe prevention of a rash. Any of the prior art documents use an approach of combining cashew nut shell liquid with a feed and diluting the liquid for administration, and do not describe an approach of administering cashew nut shell liquid to a subject animal by forced oral administration without diluting.

Patent Document 7 describes a method for reducing a rash, comprising adding at least one of an oil absorbent and porous powder to cashew nut shell liquid to solidify the cashew nut shell liquid, thereby reducing a rash. Patent Document 8 describes a formulation which is produced by adsorbing cashew nut shell liquid to an oil absorbent; granulating the resultant product, and coating the surfaces of the resultant particles with a coating agent. None of the prior art documents, however, improves oral administration of cashew nut shell liquid by administration of a suspension of coated granules in a gel.

### PRIOR ART DOCUMENTS

### PATENT DOCUMENTS

Patent Document 1: JP 2008-144171 A
Patent Document 2: JP 2006-111839 A
Patent Document 3: JP 2003-252893 A
Patent Document 4: JP 2003-238400 A
Patent Document 5: JP 2001-151675 A
Patent Document 6: JP 8-231410 A
Patent Document 7: JP 2010-59070 A
Patent Document 8: International Patent WO 2011/013592 A

### SUMMARY OF THE INVENTION

The present invention is directed to provide a formulation containing cashew nut shell liquid suitable for oral administration. The present invention is also directed to prevent a rash caused by cashew nut shell liquid, anacardic acid, cardanol, or cardol during distribution and use.

Through their extensive research to solve the problems described above, the present inventors have found that a process of coating granules which contain cashew nut shell liquid and suspending the coated granules in a water-soluble gel can delay disintegration of the granules which contain cashew nut shell liquid and elution of the cashew nut shell liquid from the granules and thus can reduce the risk of a rash caused by the cashew nut shell liquid.

In this way, the present inventors have completed the present invention.

The present invention relates to:
(1) a formulation which comprises coated granules and a water-soluble gel, wherein the coated granules comprise an oil absorbent which comprises cashew nut shell liquid, anacardic acid, cardanol, or cardol, and wherein the coated granules are suspended in the water-soluble gel;
(2) the formulation according to (1), wherein elution of the cashew nut shell liquid, the anacardic acid, the cardanol, or the cardol from inside the coated granules in the water-soluble gel is delayed by 10 minutes or more, compared with a formulation which comprises uncoated granules comprising cashew nut shell liquid, anacardic acid, cardanol, or cardol and which does not include a water-soluble gel;
(3) the formulation according to (1) or (2), wherein the water-soluble gel comprises a gelling agent in an amount of 0.01-10.0% by weight;
(4) the formulation according to any one of (1)-(3), wherein the coated granules are coated with carboxymethyl cellulose, and wherein the water-soluble gel comprises carboxymethyl cellulose;
(5) the formulation according to any one of (1)-(3), wherein the coated granules are coated with α-starch, and wherein the water-soluble gel comprises carboxymethyl cellulose;
(6) the formulation according to any one of (1)-(3), wherein the coated granules are coated with carboxymethyl cellulose, and wherein the water-soluble gel comprises xanthan gum;
(7) the formulation according to any one of (1)-(3), wherein the coated granules are coated with α-starch, and wherein the water-soluble gel comprises xanthan gum;
(8) a method for producing a formulation which comprises cashew nut shell liquid, anacardic acid, cardanol, or cardol, comprising adsorbing the cashew nut shell liquid, the anacardic acid, the cardanol, or the cardol to an oil absorbent, granulating the resultant product, coating the surface of the resultant particles with a coating agent, and suspending the coated granules in a water-soluble gel;
(9) coated granules produced by adsorbing cashew nut shell liquid, anacardic acid, cardanol, or cardol to an oil absorbent, granulating the resultant product, and coating the surface of the resultant particles with a water-soluble coating agent having a molecular weight of 1000 or more;
(10) the coated granules according to (9), wherein the coating agent is carboxymethyl cellulose; and
(11) the coated granules according to (9), wherein the coating agent is α-starch.

The formulation of the present invention can prevent a rash, because the granules containing cashew nut shell liquid, anacardic acid, cardanol, or cardol are coated.

The formulation of the present invention can provide improved oral administration and facilitate administration of the granules, because suspension of the granules (solids) in a water-soluble gel allows the granules to be administered in a liquid form.

The formulation of the present invention can prevent a rash upon administration, because suspension of the coated granules in a water-soluble gel allows adjustment of the elution time of the cashew nut shell liquid.

### MODE FOR CARRYING OUT THE INVENTION

The formulation of the present invention comprises coated granules and a water-soluble gel, the coated granules being suspended in the water-soluble gel. The granules contained in the formulation of the present invention contain cashew nut shell liquid, anacardic acid, cardanol, or cardol, and an oil absorbent and are coated with a coating agent. The granules of the present invention may contain a stabilizer and/or an antioxidant for the cashew nut shell liquid.

The cashew nut shell liquid is oily liquid contained in the shell of the seed of a cashew nut obtained from the cashew nut tree (*Anacardium occidentale L.).* The cashew nut shell liquid contains, as the components thereof, anacardic acid, cardanol, and cardol. Usually, the anacardic acid is converted into cardanol by heat treatment.

Unheated cashew nut shell liquid extracted by compressing the shell of a cashew nut contains 55-80% by mass of anacardic acid, 5-20% by mass of cardanol, and 5-30% by mass of cardol, as described in J. Agric. Food Chem. 2001, 49, 2548-2551.

Heated cashew nut shell liquid obtained by heat treatment of unheated cashew nut shell liquid at 130°C or higher contains 0-10% by mass of anacardic acid, 55-80% by mass of cardanol, and 5-30% by mass of cardol, as the anacardic acid which is a main component of the unheated cashew nut shell liquid is converted into cardanol by decarboxylation.

The cashew nut shell liquid can be obtained as a vegetable oil extracted by compressing the shell of a cashew nut. The cashew nut shell liquid can also be obtained by extracting, for example, solvent-extracting a cashew nut shell. And the cashew nut shell liquid can be obtained by the process described in JP 8-231410 A such as, for example, solvent extraction.

The cashew nut shell liquid may be a commercially-available product.

The cashew nut shell liquid of the present invention may be heated cashew nut shell liquid obtained by heating unheated cashew nut shell liquid obtained as described above to 70°C or higher, preferably 130°C or higher.

The cashew nut shell liquid of the present invention may be obtained by extracting liquid (unheated cashew nut shell liquid) by compressing cashew nut shells and heating the liquid to 130°C.

The formulation of the present invention may contain anacardic acid, cardanol, or cardol, instead of cashew nut shell liquid.

The coated granules of the present invention contain cashew nut shell liquid in an amount of 15-70% by mass, preferably 18-65% by mass, more preferably 20-60% by mass, still more preferably 25-60% by mass, and most preferably 25-55% by mass, based on the total mass of the coated granules. Inclusion of cashew nut shell liquid in an amount of 15% by mass or more can effectively provide, for example, an effect of improving rumen fermentation, an effect of preventing a bloat, an effect of treating a bloat, an effect of treating acidosis, an effect of controlling a disease caused by *Clostridium,* an effect of controlling a disease caused by *Coccidia,* an effect of an agent for increasing milk yields in ruminants, an effect of an agent for preventing or treating abomasal displacement, an effect of an agent for improving the reproductivity of livestock, and an effect of increasing body weight gain of livestock. It is preferred to include cashew nut shell liquid in an amount of 70% by mass or less, because hand rash can be prevented, and the handleability of the coated granules can be maintained.

Examples of the anacardic acid used in the present invention include natural and synthetic anacardic acids and derivatives thereof. A commercial anacardic acid may also be used. The anacardic acid can be obtained by extracting cashew nut shell liquid from cashew nut shells with an organic solvent, as described in JP 8-231410 A, and eluting the anacardic acid with varying ratios of a solvent combination of n-hexane, ethyl acetate, and acetic acid, using, for example, silica gel column chromatography (see, for example, JP 3-240721 A and JP 3-240716 A). Such anacardic acid can be included in the coated granules in a similar amount to the cashew nut shell liquid.

Examples of the cardanol used in the present invention include natural and synthetic cardanols and derivatives thereof. The cardanol used in the present invention can be obtained by decarboxylating anacardic acid which is a main component of cashew nut shell liquid. Such cardanol can be included in the coated granules in a similar amount to the cashew nut shell liquid.

When heated cashew nut shell liquid is used, the heated cashew nut shell liquid preferably contains anacardic acid and cardanol in a mass ratio of 0:100-20:80.

Examples of the cardol used in the present invention include natural cardols (including cardols obtained by squeeze and distillation) and synthetic cardols and derivatives thereof. The cardol used in the present invention can also be obtained by purification of cashew nut shell liquid. Such cardol can be included in the coated granules in a similar amount to the cashew nut shell liquid.

The coated granules of the present invention contain an oil absorbent, and the cashew nut shell liquid, the anacardic acid, the cardanol, or the cardol is absorbed to and contained in the oil absorbent. The coated granules of the present invention may contain cashew nut shells and an auxiliary material, in addition to the oil absorbent.

Examples of the oil absorbent and the auxiliary material used in the present invention include silicic acid and salts thereof (such as silica), vermiculite, diatomaceous earth, talc, kaolin, bentonit, starch, α-starch, dextrin, carboxymethyl cellulose, glucose, sucrose, lactose, and trehalose. The oil absorbent and the auxiliary material are preferably in a particle form, although they may be in any form as long as they can release the cashew nut shell liquid, the anacardic acid, the cardanol, or the cardol into a rumen. Two or more of the auxiliary materials may be used in combination.

The granules of the present invention may contain a stabilizer and/or an antioxidant for cashew nut shell liquid. The stabilizer for cashew nut shell liquid used in the present invention refers to a chelating agent which is a multidentate ligand forming coordinate bonds with a metal ion in cashew nut shell liquid to produce a chelate compound.

Examples of the chelating agent used in the present invention include organic acid chelating agents, organic acid salt chelating agents, phosphoric acid chelating agents, phosphate chelating agents, amino polycarboxylic acid chelating agents, amino polycarboxylate chelating agents, neutral amino acid chelating agents, aluminosilicate chelating agents, phosphonic acid chelating agents, phosphonate chelating agents, and polymer chelating agents.

Examples of the organic acid chelating agents and the organic acid salt chelating agents include citric acid and salts thereof, malic acid and salts thereof, tartaric acid and salts thereof, succinic acid and salts thereof, gluconic acid and salts thereof, oxalic acid and salts thereof, glycolic acid and salts thereof, and propionic acid and salts thereof.

Examples of the phosphoric acid chelating agents and the phosphate chelating agents include orthophosphoric acid and salts thereof, pyrophosphoric acid and salts thereof, tripolyphosphoric acid and salts thereof, tetrapolyphosphoric acid and salts thereof, hexametaphosphoric acid and salts thereof, and phytic acid and salts thereof. Examples of the salts of orthophosphoric acid include sodium dihydrogen phosphate, potassium dihydrogen phosphate, ammonium dihydrogen phosphate, disodium hydrogen phosphate, dipotassium hydrogen phosphate, and diammonium hydrogen phosphate. Examples of the amino polycarboxylic acid chelating agents and the amino polycarboxylate chelating agents include ethylenediamine tetraacetic acid (EDTA) and salts thereof, ethylenediamine diacetic acid and salts thereof, hydroxyethyl ethylenediamine tetraacetic acid and salts thereof, diethylenetriamine pentaacetic acid and salts thereof, nitrilotriacetic acid and salts thereof, triethylenetetraamine hexaacetic acid and salts thereof, dicarboxymethyl glutamine hexaacetic acid and salts thereof, dicarboxymethyl glutamic acid tetrasodium salt, dihydroxymethyl glycine, 1,3-propanediaminetetraacetic acid and salts thereof, 1,3-diamino-2-hydroxypropane tetraacetic acid and salts thereof, phosphonobutane tricarboxylic acid and salts thereof, glutamic acid and salts thereof, cyclohexanediamine tetraacetic acid and salts thereof, iminodiacetic acid and salts thereof, N-(2-hydroxyethyl)iminodiacetic acid and salts thereof, N-(2-hydroxyethyl)ethylenediamine triacetic acid and salts thereof, glycol ether diaminetetraacetic acid and salts thereof, glutamic acid diacetic acid and salts thereof, aspartic acid diacetic acid and salts thereof, and dihydroxymethyl glycine.

Examples of the neutral amino acid chelating agents include glycine, alanine, leucine, cysteine, methionine, asparagine, and glutamine.

An example of the aluminosilicate chelating agents includes zeolite.

Examples of the phosphonic acid chelating agents and the phosphonate chelating agents include hydroxyethylidene diphosphonic acid and salts thereof, nitrilotris methylene phosphonic acid and salts thereof, and nitrilotris and salts thereof.

Examples of the polymer chelating agents include polyacrylic acid, polymaleic acid, and a copolymer of maleic acid and acrylic acid. Two or more of the chelating agents may be used in combination.

Examples of the antioxidant for cashew nut shell liquid used in the present invention include ethoxyquin, t-butylhydroxytoluene, t-butylhydroxyanisole, t-butylhydroquinone, ascorbic acid and esters thereof, vitamin E, gallic acid and esters thereof, erythorbic acid, chlorogenic acid, sulfites, thiosulfates, phosphites, hypophosphites, and phosphates. Two or more of the antioxidants may be used in combination.

The coated granules of the present invention can be obtained, for example, in the following manner.

The oil absorbent (and the auxiliary material where necessary) and the cashew nut shell liquid (and the stabilizer and/or the antioxidant where necessary) for the granules can be mixed and then granulated using a usual extrusion granulator. The granulated mixture is compressed into tablets, where necessary.

The mixture of the oil absorbent and the cashew nut shell liquid can also be compressed into tablets using a briquetting machine.

After the granulation, the pellets can be shaped to round the edges of the pellets. The shaping can be performed using, for example, a marumerizer from Dalton Co., Ltd.

In the present invention, after the granulation and, where necessary, the shaping, the pellets can be coated with, for example, a coating agent to produce the coated granules of the present invention.

The coated granules obtained as described above can be suspended in a water-soluble gel to produce the formulation of the present invention.

Examples of the coating agent used in the present invention include carboxymethyl cellulose (CMC), starch, α-starch, dextrin, hydrogenated oils, glucomannan, gelatin, agar, carrageenan, xanthan gum, guar gum, tara gum, locust bean gum, gellant gum, pectin, alginates, sodium caseinate, propylene glycol, and sodium polyacrylate. The carboxymethyl cellulose and the α-starch are preferred.

The coating agent may be water-soluble or water-insoluble. The water-soluble coating agent used in the present invention is preferably a polymer or an oligomer having a molecular weight of 1,000 or more, more preferably a molecular weight of from 1,000 to 10,000,000.

When the granules are coated with the coating agent, the granules can be coated using a tumbling granulator. In particular, the following process is performed after the granulation.
- An aqueous or an ethanol solution of the coating agent is sprayed onto the particles, and then an appropriate amount of the coating agent (in a powder form) is added and dried.
- The coating procedure is repeated to adjust the thickness.

The coated granules of the present invention preferably contain the particles and the coating agent in a mass ratio of 60-99.9:40-0.01 and more preferably 70-99.9:30-0.1. Two or more of the coating agents may be coated to form plural layers, or a combination of two or more of the coating agents may be used at the same time.

The coated granules obtained as described above can be suspended in a water-soluble gel produced from a gelling agent to obtain the formulation of the present invention. Suspension of the coated granules in the water-soluble gel allows adjustment of dispensing of the formulation of the present invention from a container such as a bottle upon administration and elution of the cashew nut shell liquid, the anacardic acid, the cardanol, or the cardol from the coated granules. As used herein, the term "dispense (dispensing)" refers to provision of the gelled granules from a container such as a bottle, and the term "elute (elution)" refers to provision of the cashew nut shell liquid, the anacardic acid, the cardanol, or the cardol from the coated granules.

Examples of the gelling agent used in the present invention include carboxymethyl cellulose, glucomannan, gelatin, starch, α-starch, agar, carrageenan, xanthan gum, guar gum, tara gum, locust bean gum, gellant gum, pectin, alginates, sodium caseinate, propylene glycol, and sodium polyacrylate. Two or more of the gelling agents may be used in combination.

The water-soluble gel of the present invention can be produced by adding water to the gelling agent as described above and optionally adding, for example, an alkali.

As used herein, the term "water-soluble gel" refers to a gel which has some degree of flowability and, for example, which can be administered as a fluid to an animal, rather than a solid-like gel which loses flowability.

The formulation of the present invention contains the gelling agent at a concentration of 0.01-10.0% by weight and preferably 0.02-5.0% by weight.

Although the formulation of the present invention may contain any combination of the coated granules and the gelling agent, the formulation preferably contains a combination of carboxymethyl cellulose and carboxymethyl cellulose, a combination of carboxymethyl cellulose and xanthan gum, a combination of α-starch and carboxymethyl cellulose, or a combination of α-starch and xanthan gum.

An inorganic dispersing agent such as silicon dioxide, magnesium oxide, aluminum oxide, iron oxide, and calcium oxide may be added depending on the use. Two or more of the dispersing agents may be used in combination.

The formulation of the present invention is a mixture of the coated granules and the water-soluble gel. Any mixing form may be used, and a necessary amount of the coated granules and a necessary amount of the water-soluble gel may be mixed previously or immediately before use to administer the formulation to an animal. The weight ratio between the coated granules and the water-soluble gel is preferably 1:2-1:50, more preferably 1:3-1:10. The present invention includes a formulation in which the coated granules and the water-soluble gel are separately provided and mixed at use.

The formulation of the present invention can be orally administered to an animal such as cattle, a goat, and sheep, as a formulation in which the coated granules and the water-soluble gel are mixed in a container such as a bottle at use or previously mixed. The formulation may be administered to a calf, a kid goat, and a lamb.

The formulation of the present invention is suitably used as an agent for improving rumen fermentation for a ruminant, an agent for preventing a bloat, an agent for treating a bloat, an agent for increasing milk yieds in a ruminant, an agent for preventing or treating abomasal displacement, an agent for improving the reproductivity of livestock, an agent for treating acidosis, an agent for controlling a disease caused by *Clostridium,* an agent for controlling a disease caused by *Coccidia,* and an agent for increasing body weight gain of livestock.

The cashew nut shell liquid, the anacardic acid, the cardanol, or the cardol may be used with an antibiotic.

Specific examples of the antibiotic include zinc bacitraci, avilamycin, alkyl trimethyl ammonium calcium oxytetracycline, efrotomycin, enramycin, chlortetracycline, sedecamycin, semduramicin, narasin, nosiheptide, virginiamycin, bicozamycin, flavophospholipol, polynactin, monensin, salinomycin, lasalocid, lysocellin, lonomycin, avoparcin, colistin sulfate, tylosin phosphate, amprolium ethopabate, amprolium ethopabate sulfaquinoxaline, morantel citrate, decoquinate, nicarbazin, halofuginone polystyrene sulfonate, kitasamycin, tiopeptone, destomycin A, and hygromycin B, and salts thereof.

### EXAMPLES

### Production Example

500 kg of cashew nut shells were obtained from Cashew Co., Ltd. and compressed to produce 158 kg of cashew nut shell liquid (unheated CNSL).

The composition of the CNSL was determined in the manner as described below. An HPLC system (Waters 600, Nihon Waters K.K.), a detector (Waters 490E, Nihon Waters K.K.), a printer (Chromatopac C-R6A, Shimadzu Corp.), and a column (Supelcosil LC18, Supelco, Inc.) were used. The analysis was carried out under the conditions of a solvent composition of acetonitrile: water: acetic acid = 80:20:1 (based on volume), a flow rate of 2 ml/min, and an absorbance of 280 nm.

The unheated cashew nut shell liquid contained 61.8% by mass of anacardic acid, 8.2% by mass of cardanol, and 19.9% by mass of cardol.

### Example 1 (Preparation of Samples)

(1) The following carboxymethyl cellulose (CMC) available from Daicel FineChem Ltd. was used.
   Item No. 1110, 1220, 1350, 2260, 2280, and 2252
(2) Xanthan gum, tara gum, and mannan available from Ina Food Industry Co., Ltd. were used.
(3) Cashew nut shell liquid
   As described in the Production Example, cashew nut shells were obtained from Cashew Co., Ltd. and compressed to produce cashew nut shell liquid.
(4) Production of granules
   2.0 kg of the cashew nut shell liquid, 1.7 kg of silica (Sipernat 22, Evonik Japan Co., Ltd.), 1.0 kg of α-starch (Corn Alpha Y, Sanwa Starch Co., Ltd.), and 1.5 kg of water were added into a Henschel mixer, and then mixed by stirring.
   The mixture was granulated using an extrusion-based granulator (Dalton Co., Ltd.) to produce granules having a diameter of 2 mm. Then the granules were shaped using a marumerizer and dried using a fluid bed drier.
(5) Production of coated granules
   The granules produced in (4) above were coated. Even when a different coating agent was used, the granules were coated in the same manner. Thus, the coating procedure when CMC Item No. 1110 was used is described below as a representative example.

A tumbling granulator was used to coat the granules. 500 g of the granules produced in (4) above were added into a tumbling granulator with a disc angle of 45°, and 50 g of the CMC was added in three portions with humidification by misting. After completion of the addition, the granules were humidified by misting to smooth the surfaces. After drying, coated granules were obtained.

### Example 2 (Evaluation of Elution of Cashew Nut Shell Liquid)

10 g of the coated granules produced in Example 1 (5) were added into a 200 ml beaker. Then 100 ml of water or a gel solution at a prescribed concentration was added and stirred. The resultant mixture was allowed to stand. Elution time was determined as the time in which the cashew nut shell liquid begins to elute from the surface of the coated granules by visual inspection.

The results when CMC Item No. 1110 was used as the coating agent are shown in Table 1 below.

**[Table 1]**

| | Coating Agent | Gel Solution | | Elution Time (min) |
|---|---|---|---|---|
| | | Gelling Agent | Concentration (% by weight) | |
| Comparative Example | None | None | - | 5 |
| Example 2 | CMC Item No. 1110 | None | - | 10 |
| | | CMC 2252 | 0.1 | 15 |
| | | | 0.5 | 24 |
| | | | 1.0 | 25 |
| | | | 1.5 | 30 |
| | | Xanthan Gum | 0.02 | 15 |
| | | | 0.04 | 20 |
| | | | 0.1 | 21 |
| | | | 0.4 | 25 |

When the uncoated granules were added to water, the elution time of the cashew nut shell liquid was 5 minutes. When the coating agent was added to water, the elution time was prolonged to 10 minutes.

On the other hand, when the coating agent was added to the gel solution, the elution time was further prolonged. When 0.1% by weight of CMC 2252 or 0.02% by weight of xanthan gum was used, the elution time was 15 minutes. When the gelling agent was used at a higher concentration than these concentrations, the tendency for the elution time to be prolonged was confirmed.

### Example 3 (Evaluation of Elution of Cashew Nut Shell Liquid)

The results when CMC Item No. 1350 was used as the coating agent are shown in Table 2 below.

**[Table 2]**

| | Coating Agent | Gel Solution | | Elution Time (min) |
|---|---|---|---|---|
| | | Gelling Agent | Concentration (% by weight) | |
| Comparative Example | None | None | - | 5 |
| Example 3 | CMC Item No. 1350 | None | - | 11 |
| | | CMC 2252 | 0.1 | 16 |
| | | | 0.5 | 24 |
| | | | 1.0 | 27 |
| | | | 1.5 | 30 |
| | | Xanthan Gum | 0.02 | 15 |
| | | | 0.04 | 22 |
| | | | 0.1 | 22 |
| | | | 0.4 | 26 |

When the coated granules were added to water, the elution time was 11 minutes. When the coated granules were added to the gel solution, the elution was delayed as in Example 2. When 0.02% by weight of xanthan gum was used, the elution time was 15 minutes. When 0.1% by weight of CMC 2252 was used, the elution time was 16 minutes. When the gelling agent was used at a higher concentration than these concentrations, the tendency for the elution time to be prolonged was confirmed, as in Example 2.

### Example 4 (Evaluation of Elution of Cashew Nut Shell Liquid)

The results when α-starch was used as the coating agent are shown in Table 3 below.

**[Table 3]**

| | Coating Agent | Gel Solution | | Elution Time (min) |
|---|---|---|---|---|
| | | Gelling Agent | Concentration (% by weight) | |
| Comparative Example | None | None | - | 5 |
| Example 4 | α-starch | None | - | 10 |
| | | CMC 2252 | 0.1 | 13 |
| | | | 0.5 | 19 |
| | | | 1.0 | 21 |
| | | | 1.5 | 25 |
| | | Xanthan Gum | 0.02 | 12 |
| | | | 0.04 | 14 |
| | | | 0.1 | 21 |
| | | | 0.4 | 23 |

When the coated granules were added to water, the elution time was 10 minutes. When the coated granules were added to the gel solution, the elution was delayed as in Example 2. When 0.02% by weight of xanthan gum was used, the elution time was 12 minutes. When 0.1% by weight of CMC 2252 was used, the elution time was 13 minutes. When the gelling agent was used at a higher concentration than these concentrations, the tendency for the elution time to be prolonged was confirmed, as in Example 2.

### Example 5 (Evaluation of Flowability of Gelled Granules)

55 g of the coated granules which contained CMC Item No. 1110 as the coating agent was added into a beer bottle (633 ml large bottle). 250 g of an aqueous solution of a respective gelling agent at a prescribed concentration was added into the bottle. The bottle was shaken to homogenize the content, and then inclined downwardly at 45° to study dispensing of the coated granules from the bottle. The results are shown in Table 4 below.

**[Table 4]**

| Gelling Agent | Concentration Range Which Allows Dispensing of Granules (% by weight) |
|---|---|
| CMC 1110 | 0.05-3.0 |
| CMC 1220 | 0.10-5.0 |
| CMC 1350 | 0.02-2.5 |
| CMC 2260 | 0.02-1.5 |
| CMC 2280 | 0.02-1.0 |
| CMC 2252 | 0.02-1.5 |
| Xanthan Gum | 0.02-0.6 |
| Tara Gum | 0.05-1.0 |
| Mannan | 0.05-2.0 |

The general trend was that a low concentration of the gelling agent caused clogging at the bottle mouth with the gelled granules, while a high concentration of the gelling agent provided a low flowability, which resulted in failure to dispense the coated granules. Table 4 shows a concentration range which allows smooth dispensing. In other words, a low concentration can prevent clogging at the bottle mouth with the gelled granules, as long as the concentration is within the range shown in Table 4, while a high concentration can allow dispensing of 90% or more of the gelled granules, as long as the concentration is within the range shown in Table 4.

### INDUSTRIAL APPLICABILITY

The formulation of the present invention produced by suspending the granules coated with the coating agent in the water-soluble gel can delay disintegration of the granules upon administration of the suspension and can reduce the risk of a rash caused by cashew nut shell liquid.

## Claims

1. A formulation which comprises coated granules and a water-soluble gel, wherein the coated granules comprise an oil absorbent which comprises cashew nut shell liquid, anacardic acid, cardanol, or cardol, and wherein the coated granules are suspended in the water-soluble gel.

2. The formulation according to claim 1, wherein elution of the cashew nut shell liquid, the anacardic acid, the cardanol, or the cardol from inside the coated granules in the water-soluble gel is delayed by 10 minutes or more, compared with a formulation which comprises uncoated granules comprising cashew nut shell liquid, anacardic acid, cardanol, or cardol and which does not include a water-soluble gel.

3. The formulation according to claim 1 or 2, wherein the water-soluble gel comprises a gelling agent in an amount of 0.01-10.0% by weight.

4. The formulation according to any one of claims 1-3, wherein the coated granules are coated with carboxymethyl cellulose, and wherein the water-soluble gel comprises carboxymethyl cellulose.

5. The formulation according to any one of claims 1-3, wherein the coated granules are coated with α-starch, and wherein the water-soluble gel comprises carboxymethyl cellulose.

6. The formulation according to any one of claims 1-3, wherein the coated granules are coated with carboxymethyl cellulose, and wherein the water-soluble gel comprises xanthan gum.

7. The formulation according to any one of claims 1-3, wherein the coated granules are coated with α-starch, and wherein the water-soluble gel comprises xanthan gum.

8. A method for producing a formulation which comprises cashew nut shell liquid, anacardic acid, cardanol, or cardol, comprising adsorbing the cashew nut shell liquid, the anacardic acid, the cardanol, or the cardol to an oil absorbent, granulating the resultant product, coating the surface of the resultant particles with a coating agent, and suspending the coated granules in a water-soluble gel.

9. Coated granules produced by adsorbing cashew nut shell liquid, anacardic acid, cardanol, or cardol to an oil absorbent, granulating the resultant product, and coating the surface of the resultant particles with a water-soluble coating agent having a molecular weight of 1000 or more.

10. The coated granules according to claim 9, wherein the coating agent is carboxymethyl cellulose.

11. The coated granules according to claim 9, wherein the coating agent is α-starch.
